# FASCICULE DE BREVET EUROPEEN

(11) **EP 2 521 738 B1**
(45) Date de publication et mention de la délivrance du brevet: **30.10.2013**
(21) Numéro de dépôt: 10807336.2
(22) Date de dépôt: 14.12.2010
(51) Int. Cl.: C08B 37/00, A61K 31/728, A61K 8/73, A61Q 19/00

(54) **COMPOSITION COSMÉTIQUE OU DERMATOLOGIQUE, PROCÉDÉ DE TRAITEMENT COSMÉTIQUE ET DÉRIVÉ D'ACIDE HYALURONIQUE**
KOSMETISCHE ODER DERMATOLOGISCHE ZUSAMMENSETZUNG, KOSMETISCHES BEHANDLUNGSVERFAHREN UND HYALURONSÄUREDERIVAT
COSMETIC OR DERMATOLOGICAL COMPOSITION, COSMETIC TREATMENT METHOD, AND HYALURONIC ACID DERIVATIVE

(30) Priorité: 04.02.2010 US 301344 P; 04.01.2010 FR 1050010
(43) Date de publication de la demande: 14.11.2012
(73) Titulaire: L'Oréal, 75008 Paris (FR)
(72) Inventeur: CHODOROWSKI-KIMMES, Sandrine, F-60300 Senlis (FR); DALKO, Maria, F-78000 Versailles (FR)
(74) Mandataire: Kromer, Christophe
(86) Numéro de dépôt international: PCT/FR2010/052713
(87) Numéro de publication internationale: WO 2011/080450

(56) Documents cités:
- WO-A1-00/16818
- WO-A1-99/56799
- WO-A2-00/27887

## Description

La présente invention concerne une composition cosmétique ou dermatologique comprenant des dérivés fonctionnalisés préparés à partir d'acide hyaluronique ainsi qu'un procédé de traitement cosmétique employant ladite composition.

Les femmes et les hommes ont tendance actuellement à vouloir paraître jeunes le plus longtemps possible et cherchent par conséquent à estomper les marques du vieillissement de la peau, telles que la perte de fermeté, d'élasticité, de tonicité et/ou de souplesse de la peau et la formation des rides et des ridules. A ce sujet, la publicité et la mode font état de produits destinés à garder le plus longtemps possible une peau éclatante et sans rides, marques d'une peau jeune, d'autant plus que l'aspect physique agit sur le psychisme et/ou sur le moral.

La peau humaine est constituée de deux compartiments à savoir un compartiment superficiel, l'épiderme, et un compartiment profond, le derme.

L'épiderme est en contact avec l'environnement extérieur. Son rôle consiste à protéger l'organisme de la déshydratation et des agressions extérieures, qu'elles soient chimiques, mécaniques, physiques ou infectieuses. L'épiderme humain naturel est composé principalement de trois types de cellules : les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire. On y trouve aussi des leucocytes, des mastocytes ou encore des macrophages tissulaires. Il est également traversé par des vaisseaux sanguins et des fibres nerveuses.

La matrice extracellulaire du derme est composée de protéines appartenant à plusieurs grandes familles: les collagènes, les glycoprotéines matricielles autres que les collagènes (fibronectine, laminine), l'élastine, des protéoglycannes et des glycosaminoglycannes (GAGs) sous forme libre (c'est-à-dire non liés à une protéine), dont l'acide hyaluronique. Ces protéines sont majoritairement synthétisées par les fibroblastes et l'on sait que le processus de vieillissement cutané induit une diminution de ces activités métaboliques conduisant à une diminution des protéines de la matrice extracellulaire du derme et une diminution de la croissance cellulaire conduisant à une altération des propriétés mécaniques de la peau, en particulier sa fermeté, son élasticité, sa tonicité et/ou sa souplesse.

Le vieillissement cutané est un processus physiologique naturel; il n'est pas considéré comme une condition pathologique ou un désordre thérapeutique.

Les principaux signes cliniques du vieillissement cutané sont notamment l'apparition de ridules et/ou de rides, en augmentation avec l'âge. Ces rides peuvent être profondes, moyennes ou superficielles, et touchent en particulier les sillons nasogéniens, la zone périorbitaire, le contour des lèvres, le front (ride du lion); ces rides et ridules se traduisent par une dépression ou des sillons à la surface de la peau.

Différentes méthodes ont été proposées pour lutter contre les rides et les ridules, par lesquelles l'utilisation de produits de soin de la peau contenant des actifs cosmétiques (antirides, hydratants, tenseur notamment). A cette fin, l'acide hyaluronique a été proposé comme actif susceptible d'être utilisé dans de nombreuses applications cosmétiques, notamment comme actif anti-âge. Il joue également un rôle important dans l'hydratation et l'élasticité de la peau. L'acide hyaluronique utilisé dans les formulations cosmétiques se présente généralement sous la forme de hyaluronate de sodium.

Afin d'augmenter son efficacité, il a également été proposé de l'utiliser en injection, par mésothérapie ou comme produit de comblement des rides.

Toutefois, on a constaté que cet actif avait une durée de vie limitée sur la peau, mais tout particulièrement lorsqu'il était injecté. En effet, les hyaluronidases sont des enzymes présentes dans la peau qui dégradent l'acide hyaluronique et donc en diminuent l'impact dans les compositions cosmétiques. La dégradation de l'acide hyaluronique se fait grâce à l'action combinée de 3 différentes hyaluronidases. La demi-vie de l'acide hyaluronique, due au catabolisme très rapide de la molécule, varie d'un tissu à l'autre; à titre indicatif, elle est d'environ un jour au niveau dermique et épidermique.

Du fait de sa courte demi-vie, il a été proposé de réticuler l'acide hyaluronique. La réticulation peut se faire en milieu alcalin, en utilisant les sites carboxyliques et hydroxyles de la molécule. Mais cette réticulation peut également être réalisée en milieu acide, bien que les liaisons créées dans ce milieu soient nettement moins résistantes que celles créées en milieu alcalin. Le procédé de réticulation ne change pas le caractère polyanionique du polysaccharide, mais diminue sensiblement la miscibilité à l'eau du gel obtenu. Parmi les différents réticulants susceptibles d'être utilisés, on peut citer la divinylsulfone le 1,2,7,8-diépoxyoctane et le 1,4-butanedioldiglycidyléther.

Toutefois, dans certains cas, il peut être préférable de disposer d'acide hyaluronique non réticulé, notamment lorsqu'il est prévu de l'injecter par exemple dans l'épiderme ou de l'employer par mésothérapie; toutefois, on souhaite également qu'il soit quand même résistant à la dégradation enzymatique. On a constaté que les modifications chimiques pouvaient affecter les caractéristiques physico-chimiques et les propriétés biologiques de l'acide hyaluronique, ainsi que son devenir après application; par ailleurs, la demi-vie des dérivés d'acide hyaluronique, et donc leur biodisponibilité, reste toujours trop courte.

La présente invention a pour but de proposer des composés dérivés d'acide hyaluronique, pouvant être réticulés ou non réticulés, mais présentant une bonne résistance à la dégradation enzymatique.

Ces dérivés trouvent une application toute particulière dans les compositions cosmétiques permettant de prévenir et/ou diminuer les effets du vieillissement et/ou de restaurer les propriétés mécaniques de la peau telles que sa fermeté, son élasticité et/ou sa souplesse.

Un objet de l'invention est donc une composition cosmétique ou dermatologique comprenant un composé qui comprend des motifs (Ia) et (Ib) tels que définis ci-après. Dans la suite de la présente description, ces composés sont appelés 'dérivés d'acide hyaluronique'.

Lesdits dérivés d'acide hyaluronique forment un autre objet de l'invention.

On a constaté que la fonctionnalisation de l'acide hyaluronique par des entités capables de former 4 liaisons hydrogène, plus spécifiquement la fonctionnalisation par des uréidopyrimidones, permettait de résoudre le problème de dégradation enzymatique.

Par ailleurs, les propriétés mécaniques des dérivés ainsi préparés peuvent être modulées, notamment en fonction du taux de fonctionnalisation.

L'acide hyaluronique appartient à la famille des glycosaminoglycannes (GAGs). Il est formé par la répétition d'unité disaccharidique hydrophile, dans laquelle le D-glucuronate de sodium et/ou l'acide D-glucuronique est relié à la N-acétylglucosamine par des liaisons glycosidiques alternées β1-4 et β1-3 ou β1-4 seules. En condition physiologique, ce polysaccharide se trouve non sous forme acide mais sous la forme d'un sel sodique, le hyaluronate de sodium. Un certain nombre de dérivés de l'acide Hyaluronique sont mentionnés dans les Brevets WO 00/27887, WO 99/56799 et WO 00/16818.

Par acide hyaluronique, on entend dans la présente description l'acide hyaluronique ou l'un de ses sels, en particulier ses sels de sodium, potassium, magnésium et calcium.

L'acide hyaluronique, d'une manière générale, comprend donc des motifs de répétition de formule (la) telle que décrit ci-après.

Dans les dérivés selon l'invention, une partie de ces motifs (la) a été fonctionnalisée par un groupement comprenant un motif uréidopyrimidone de formule (I), ce qui conduit à des motifs fonctionnalisés répondant à la formule (Ib) telle que décrite ci-après.

Les dérivés selon l'invention comprennent donc à la fois des motifs (la) et (Ib) : 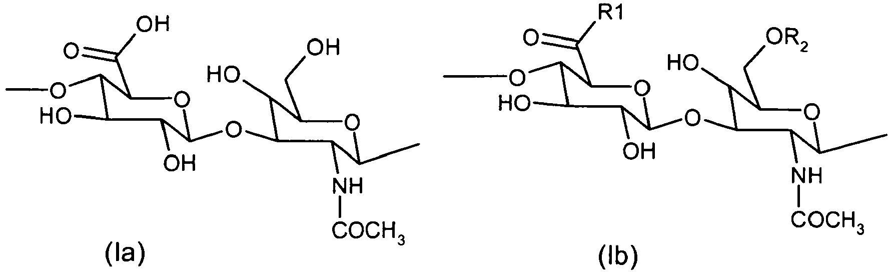 dans lesquels :
- R1 représente OH ou un radical -NH-R'-Z,
- R2 représente H ou un radical -C(O)-NH-R'-Z,
   avec :
- R' est un radical alkyle divalent, linéaire ou ramifié, en C1-C18, notamment C2-C14, voire C4-C10; ou une liaison simple;
- Z est un radical de formule (I) : 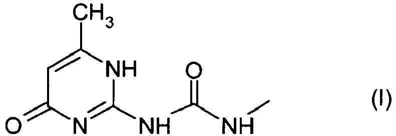
étant entendu qu'au moins l'un, de préférence un seul, des radicaux R1 et R2 comprend un radical Z.

De préférence, le rapport pondéral entre les motifs (la) et les motifs (Ib) est tel que le taux de fonctionnalisation du dérivé est compris entre 1 et 30%.

Les dérivés selon l'invention ont un poids moléculaire moyen (Mw) compris entre
50 000 et 2 000 000 daltons.

Il est rappelé que les dérivés selon l'invention peuvent se présenter sous forme de sels, notamment de sel de sodium, d'ammonium ou de potassium.

Les dérivés d'acide hyaluronique selon l'invention trouvent une application toute particulière dans les compositions cosmétiques ou dermatologiques.

Ils peuvent être présents dans lesdites compositions, seul ou en mélange, en une quantité allant de 0,1 à 3% en poids par rapport au poids total de la composition, en particulier de 0,5 à 2% en poids, voire de 0,8 à 1,5% en poids.

Les compositions cosmétiques ou dermatologiques selon l'invention comprennent par ailleurs un milieu cosmétiquement ou dermatologiquement acceptable, c'est-à-dire compatible avec les matières kératiniques telles que la peau du visage ou du corps, les lèvres, les cheveux, les cils, les sourcils et les ongles.

Les compositions selon l'invention peuvent se présenter sous toutes les formes galéniques classiquement utilisées, notamment pour une application topique, et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique (liposomes, niosomes, oléosomes), de nanoémulsions, ou de films fins. Ces compositions sont préparées selon les méthodes usuelles.

Les compositions selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

La composition selon l'invention peut notamment se présenter sous la forme d'un produit de soin ou de maquillage de la peau du visage ou du corps, des lèvres, des ongles, des cils, des sourcils, des cheveux; d'un gel ou lotion après-rasage; d'une crème dépilatoire; d'une composition d'hygiène, corporelle ou capillaire, telle qu'un gel douche, un shampooing, un savon, un pain de nettoyage; d'un produit capillaire, notamment de nettoyage, de coiffage, de soin, de traitement, de réparation, de coloration, de fixation des cheveux, tel qu'une lotion de mise en plis, une crème ou d'un gel coiffant, une composition de teinture, une lotion restructurante pour cheveux, une composition de permanente, une lotion ou gel antichute; ou encore d'une composition à usage bucco-dentaire.

Le milieu physiologiquement acceptable dans lequel les composés peuvent être employés, ainsi que ses constituants, leur quantité, la forme galénique de la composition et son mode de préparation, peuvent être choisis par l'homme du métier sur la base de ses connaissances générales en fonction du type de composition recherchée.

Notamment, la composition peut comprendre tout corps gras usuellement utilisé dans le domaine d'application envisagé. On peut notamment citer les corps gras siliconés tels que les huiles, les gommes et les cires de silicone, ainsi que les corps gras non siliconés tels que les huiles, les pâteux et les cires d'origine végétale, minérale, animale et/ou synthétique. Les huiles peuvent éventuellement être volatiles ou non volatiles.

La composition peut également comprendre un milieu aqueux qui comprend de l'eau, un milieu hydroalcoolique contenant un alcool en C2-C6 tel que l'éthanol ou l'isopropanol, ou un milieu organique comprenant des solvants organiques usuels tels que des alcools en C2-C6, notamment l'éthanol et l'isopropanol, des glycols tels que le propylène glycol, des cétones.

La composition selon l'invention peut également comprendre les adjuvants habituels dans les domaines cosmétique et dermatologique, tels que les épaississants, les émulsionnants, les tensioactifs, les gélifiants, les actifs cosmétiques, les parfums, les charges, les matières colorantes, les hydratants, les vitamines, les polymères. Les quantités de ces différents adjuvants sont celles classiquement utilisées dans les domaines considérés, par exemple de 0,001 à 20 % du poids total de la composition. Ces adjuvants, ainsi que leurs concentrations, doivent être tels qu'ils ne nuisent pas aux propriétés avantageuses des composés selon l'invention.

L'invention a également pour objet un procédé de traitement cosmétique, notamment de maquillage, de soin, de nettoyage, de coloration, des matières kératiniques, notamment de la peau du corps ou du visage, des lèvres, des ongles, des cheveux et/ou des cils, comprenant l'utilisation, notamment l'application sur lesdites matières kératiniques, d'une composition cosmétique telle que définie ci-dessus.

De préférence, il s'agit d'un procédé de traitement cosmétique pour diminuer les signes du vieillissement de la peau et/ou des muqueuses, en particulier pour diminuer les rides et les ridules et/ou redonner au visage ou au corps son volume.

L'invention est illustrée plus en détails dans les exemples suivants.

### Exemple 1

La procédure utilisée pour la synthèse est celle décrite dans WO2000/016818 et représentée ci-dessous :

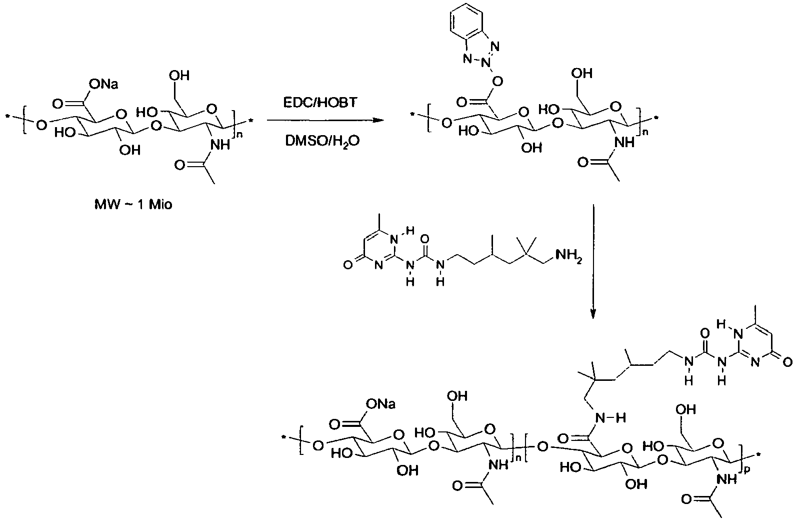

On ajoute 10 ml de 1,6-diamino-2,2,4-triméthylhexane à 2,1 g d'uréidopyrimidone fonctionnalisé imidazole (fourni par la société SupraPolix), suivi d'une agitation sous atmosphère contrôlé à température ambiante pendant 30 minutes. Après addition de diéthyléther, on obtient un précipité blanc, qui est filtré, puis redissout dans le chloroforme, suivi d'une deuxième précipitation dans le diéthyléther. Le précipité blanc est séché sous vide et utilisé tel quel pour la fonctionnalisation de l'acide hyaluronique.

On dissout 1 g d'hyaluronate de sodium dans 170 ml d'eau. On y ajoute 0,923 g du dendron uréidopyrimidone fonctionnalisé amine obtenu précédemment, en solution dans 170 ml de DMSO anhydre. La solution est mélangée à température ambiante et le pH est ajusté à 6.8 par addition d'une solution à 0.1M de NaOH. On dissout 1,34 g de 1-hydroxybenzitriazole (HOBt) dans 5 ml de DMSO et on l'additionne au mélange réactionnel, suivi de l'addition de 1,91 g de 1-éthyl-3-(3-(dimethylamino)propyl)-carbodiimine. Après agitation, le pH de la solution est maintenu à 6,8 par addition d'une solution 0.1 M de NaOH, jusqu'à stabilisation du pH. Le pH est ensuite amené à 7, et on ajoute 15 g d'une solution aqueuse de NaCl. Le mélange réactionnel est ensuite précipité dans 4 litres d'un mélange éthanol/eau (4v/1v) et laissé au repos durant la nuit. Le mélange est ensuite filtré, rincé 4 fois avec un mélange éthanol/eau(4v/1v), suivi de deux rinçages avec 200 ml d'un mélange éthanol/eau (9v/1v), et finalement avec 200 ml d'éthanol.

On obtient le produit recherché sous forme d'un solide blanc qui est séché sous vide à 40°C (rendement : 89%).

### Exemple 2 : Synthèse à partir de l'acide hyaluronique sous forme de sel de sodium

La procédure générale est représentée ci- dessous :

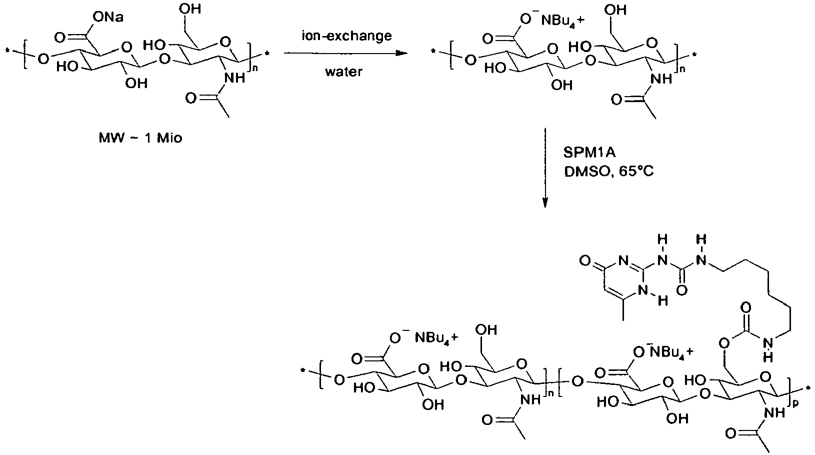

On dissout 0,5 g d'hyaluronate de sodium (polymère avec des répétitions de cycles de sucre C₁₄H₂₀NO₁₁Na, FW=401 dans 400 ml d'eau et on élue sur une colonne échangeuse d'ions Dowex 50x2-200 (13G, 4.8meq/G resin). Le pH de la solution visqueuse d'hyaluronate de sodium passe d'une valeur initiale d'environ 6 à 3-4. La solution résultante d'acide hyaluronique est neutralisée par une solution à 40% de tétrabutyl ammonium hydroxyde, afin d'avoir au final une solution avec un pH autour de 6-7.La solution est lyophilisée et permet d'obtenir de façon quantitative l'acide hyaluronique sous sa forme O⁻NBu₄⁺

On dissout 0,2 g du sel ainsi obtenu dans 10 ml de DMSO et le dendron uréidopyrimidone fonctionnalisé isocyanate (fourni par la société SupraPolix) ci-dessous est ajouté à la solution :

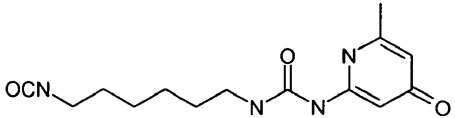

Le ratio molaire est ajusté en fonction du taux de fonctionnalisation de l'acide hyaluronique désiré. Une goutte de catalyseur dibutylétain dilaurate est ajouté au mélange réactionnel. La solution visqueuse est chauffée à 70°C, et l'agitation est maintenue pendant 12 heures sous atmosphère contrôlée. La réaction est suivie par spectroscopie infrarouge avec disparition du pic caractéristique des isocyanates. Une fois la réaction stoppée, le mélange réactionnel est dissout dans 10 ml de DMSO et filtré sur célite. La solution résultante est précipitée goutte à goutte dans 200 ml d'un mélange diéthyléther/THF (1v/1v), et le solide blanc ainsi récupéré est lavé avec un mélange diéthyléther/THF (5v/3v) puis un mélange diéthyléther/THF (3v/1v). Le rendement varie entre 50 et 100% en fonction du taux de fonctionnalisation. Afin de s'assurer que le dendron uréidopyrimidone a réagi complètement, on peut prélever 1 ml de la solution résultante et la dissoudre dans 1 ml d'eau : une solution claire nous assure de la complète réactivité du dendron.

Pour obtenir l'acide hyaluronique sous sa forme de sel de sodium, on dissout 60 mg de la poudre obtenue précédemment dans 10 ml d'eau, l'agitation étant maintenue pendant la nuit. Après deux dialyses successives dans une solution saline de NaCl, suivie de trois dialyses dans l'eau, la solution aqueuse est lyophilisée et permet d'obtenir le produit désiré sous forme de poudre blanche.

### Exemple 3 : test comparatif

### Réactifs :

- tampon d'essai : tampon phosphate 0,1 M, pH=5,3
- acide hyaluronique préparé à 1,2 mg/ml (2x) en tampon d'essai
- hyaluronidase (Sigma type IV-S, H3884) préparé à 1 mg/ml (3x) dans du tampon d'essai
- albumine bovine BSA (Sigma A7888) préparée à 1% dans tampon acétate 0,5M, pH=4,2.

Les composés des exemples 1 et 2 sont mis en présence de l'enzyme et préincubés sur glace pendant 10 minutes. On teste 5 concentrations de produit, et les traitements sont réalisés en n=3. A la fin de la pré-incubation, l'acide hyaluronique a été ajouté et une incubation de 60 minutes à 37°C a été réalisée avant précipitation de l'acide hyaluronique avec une solution de BSA.

L'absorption (DO) à 540 nm a été mesurée à l'aide d'un lecteur de plaque (Thermomax, Molecular Devices).

On constate que les composés des exemples 1 et 2 ne sont pas de substrats de hyaluronidases; ils ne sont pas dégradés par l'enzyme, contrairement à l'acide hyaluronique natif.

## Revendications

1. Composition cosmétique ou dermatologique comprenant un composé d'acide hyaluronique formé par la répétition d'unité disaccharidique hydrophile, dans laquelle le D-glucuronate de sodium et/ou l'acide D-glucuronique est relié à la N-acétylglucosamine par des liaisons glycosidiques alternées β1-4 et β1-3 ou β1-4 seules, comprenant des motifs (la) et des motifs (Ib), ou l'un de ses sels, : 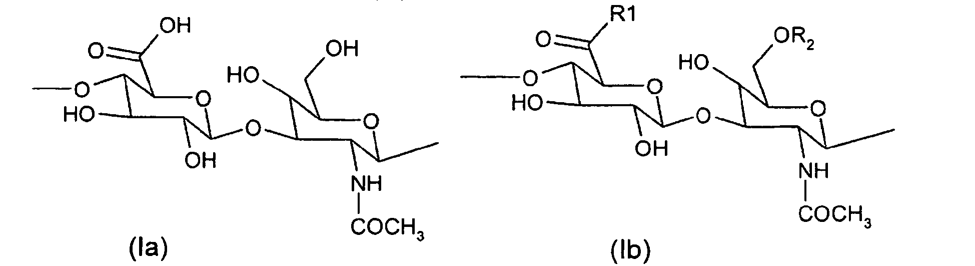 dans lesquels :
- R1 représente OH ou un radical -NH-R'-Z,
- R2 représente H ou un radical -C(O)-NH-R'-Z,
avec :
- R' est un radical alkyle divalent, linéaire ou ramifié, en C1-C18, notamment C2-C14, voire C4-C10; ou une liaison simple;
- Z est un radical de formule (I) : 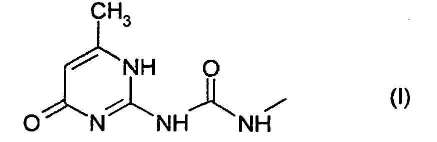
étant entendu qu'au moins l'un des radicaux R1 et R2 comprend un radical Z,
le taux de fonctionnalisation du composé étant compris entre 1 et 30 %,
le composé ayant un poids moléculaire moyen Mw compris entre 50 000 et 2 000 000.

2. Composition selon la revendication précédente, dans laquelle le composé est présent, seul ou en mélange, en une quantité allant de 0,1 à 3% en poids par rapport au poids total de la composition, en particulier de 0,5 à 2% en poids, voire de 0,8 à 1,5% en poids.

3. Composition selon l'une des revendications précédentes, se présentant sous la forme d'un produit de soin ou de maquillage de la peau du visage ou du corps, des lèvres, des ongles, des cils, des sourcils, des cheveux; d'un gel ou lotion après-rasage; d'une crème dépilatoire; d'une composition d'hygiène, corporelle ou capillaire, telle qu'un gel douche, un shampooing, un savon, un pain de nettoyage; d'un produit capillaire, notamment de nettoyage, de coiffage, de soin, de traitement, de réparation, de coloration, de fixation des cheveux, tel qu'une lotion de mise en plis, une crème ou d'un gel coiffant, une composition de teinture, une lotion restructurante pour cheveux, une composition de permanente, une lotion ou gel antichute; ou encore d'une composition à usage bucco-dentaire.

4. Composition selon l'une des revendications précédentes, comprenant au moins un ingrédient choisi parmi les corps gras siliconés tels que les huiles, les gommes et les cires de silicone, les corps gras non siliconés tels que les huiles, les pâteux et les cires d'origine végétale, minérale, animale et/ou synthétique; de l'eau, un alcool en C2-C6; des glycols tels que le propylène glycol, des cétones; les épaississants, les émulsionnants, les tensioactifs, les gélifiants, les actifs cosmétiques, les parfums, les charges, les matières colorantes, les hydratants, les vitamines, les polymères.

5. Procédé de traitement cosmétique, notamment de maquillage, de soin, de nettoyage, de coloration, des matières kératiniques, notamment de la peau du corps ou du visage, des lèvres, des ongles, des cheveux et/ou des cils, comprenant l'application sur les matières kératiniques d'une composition cosmétique telle que définie à l'une des revendications 1 à 4.

6. Procédé selon la revendication 5, **caractérisé en ce qu'**il s'agit d'un procédé de traitement cosmétique pour diminuer les signes du vieillissement de la peau et/ou des muqueuses, en particulier pour diminuer les rides et les ridules et/ou redonner au visage ou au corps son volume.

7. Composé d'acide hyaluronique formé par la répétition d'unité disaccharidique hydrophile, dans laquelle le D-glucuronate de sodium et/ou l'acide D-glucuronique est relié à la N-acétylglucosamine par des liaisons glycosidiques alternées β1-4 et β1-3 ou β1-4 seules, comprenant des motifs (Ia) et des motifs (Ib), ou l'un de ses sels, : 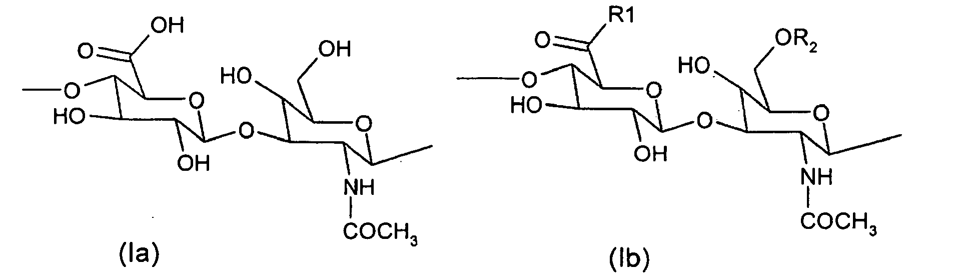 dans lesquels :
- R1 représente OH ou un radical -NH-R'-Z,
- R2 représente H ou un radical -C(O)-NH-R'-Z,
avec :
- R' est un radical alkyle divalent, linéaire ou ramifié, en C1-C18, notamment C2-C14, voire C4-C10; ou une liaison simple;
- Z est un radical de formule (I) : 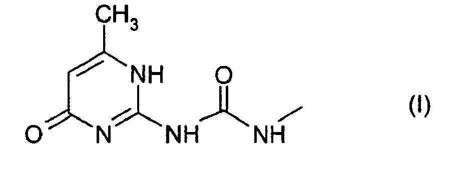
étant entendu qu'au moins l'un des radicaux R1 et R2 comprend un radical Z,
le taux de fonctionnalisation du composé étant compris entre 1 et 30 %,
le composé ayant un poids moléculaire moyen Mw compris entre 50 000 et 2 000 000.

## Patentansprüche

1. Kosmetische oder dermatologische Zusammensetzung, umfassend eine Hyaluronsäureverbindung, gebildet durch die hydrophile Disaccharid-Wiederholungseinheit, worin das Natrium-D-glucuronat und/oder die D-Glucuronsäure mit dem N-Acetylglucosamin im Wechsel durch die β1-4- und β1-3-glycosidischen Bindungen oder nur durch β1-4-glycosidische Bindungen verbunden ist, umfassend Strukturelemente (Ia) und Strukturelemente (Ib), oder eines seiner Salze: 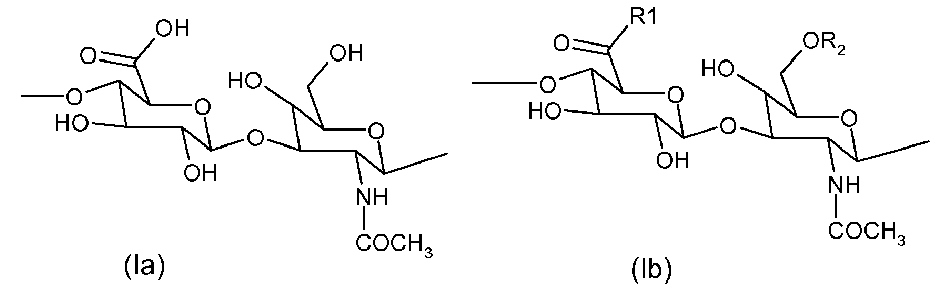 worin:
- R1 für OH oder einen Rest -NH-R'-Z steht,
- R2 für H oder einen Rest -C(O)-NH-R'-Z steht,
wobei:
- R' ein zweiwertiger, linearer oder verzweigter C1-C18-, insbesondere C2-C14-, sogar C4-C10-Alkylrest oder eine Einfachbindung ist;
- Z ein Rest mit der Formel (I) ist: 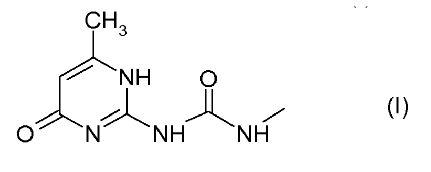
wobei als vereinbart gilt, dass mindestens einer der Reste R1 und R2 einen Rest Z umfasst,
der Funktionalisierungsgrad der Verbindung im Bereich zwischen 1 und 30 % liegt,
die Verbindung ein mittleres Molekulargewicht Mw im Bereich zwischen 50.000 und 2.000.000 aufweist.

2. Zusammensetzung nach dem vorhergehenden Anspruch, wobei die Verbindung allein oder als Gemisch in einer Menge vorhanden ist, die, bezogen auf das Gesamtgewicht der Zusammensetzung, von 0,1 bis 3 Gew.-%, insbesondere von 0,5 bis 2 Gew.-%, sogar 0,8 bis 1,5 Gew.-% reicht.

3. Zusammensetzung nach einem der vorhergehenden Ansprüche, die die Form eines Produkt zur Pflege oder zum Schminken der Haut des Gesichts oder des Körpers, der Lippen, der Nägel, der Wimpern, der Augenbrauen, der Haare; eines Gels oder einer Aftershave-Lotion; einer enthaarenden Creme; einer Körper- oder Haarpflegezusammensetzung, wie etwa eines Duschgels, eines Shampoos, einer Seife, eines Waschstücks; eines Haarprodukts, insbesondere zur Reinigung, zum Kämmen, zur Pflege, zur Behandlung, zur Reparatur, zum Färben, zur Fixierung der Haare, wie etwa einer Wellenlotion, einer Festigercreme oder eines Festigergels, einer Färbezusammensetzung, einer restrukturierenden Lotion für Haare, einer Dauerwellenzusammensetzung, einer Lotion oder eines Gels zur Bekämpfung von Haarausfall; oder auch einer Zusammensetzung zur Mund- und Zahnpflege aufweist.

4. Zusammensetzung nach einem der vorhergehenden Ansprüche, umfassend mindestens einen Bestandteil, ausgewählt aus silikonierten Fetten, wie etwa silikonierten Ölen, Gummen und Wachsen, nicht silikonierten Fetten, wie etwa Ölen, Pasten und Wachsen pflanzlichen, mineralischen, tierischen und/oder synthetischen Ursprungs; Wasser, C2-C6-Alkohol; Glykolen, wie etwa Propylenglykol, Ketonen; Eindickungsmitteln, Emulgatoren, Tensiden, Gelbildnern, kosmetischen Wirkstoffen, Parfums, Füllstoffen, Farbstoffen, Feuchtigkeitsspendern, Vitaminen, Polymeren.

5. Verfahren zur kosmetischen Behandlung, insbesondere zum Schminken, zur Pflege, zur Reinigung, zum Färben von Keratinmaterialien, insbesondere der Haut des Körpers oder des Gesichts, der Lippen, der Nägel, der Haare und/oder der Wimpern, umfassend die Anwendung einer kosmetischen Zusammensetzung, wie in einem der Ansprüche 1 bis 4 definiert, auf den Keratinmaterialien.

6. Verfahren nach Anspruch 5, **dadurch gekennzeichnet, dass** es sich um ein Verfahren zur kosmetischen Behandlung handelt, um die Zeichen der Alterung der Haut und/oder der Schleimhäute zu verringern, insbesondere um die Falten und Fältchen zu verringern und/oder dem Gesicht oder dem Körper sein Volumen zurückzugeben.

7. Hyaluronsäureverbindung, gebildet durch die hydrophile Disaccharid-Wiederholungseinheit, worin das Natrium-D-glucuronat und/oder die D-Glucuronsäure mit dem N-Acetylglucosamin im Wechsel durch die β1-4- und β1-3-glycosidischen Bindungen oder nur β1-4-glycosidische Bindungen verbunden ist, umfassend Strukturelemente (Ia) und Strukturelemente (Ib), oder eines seiner Salze: 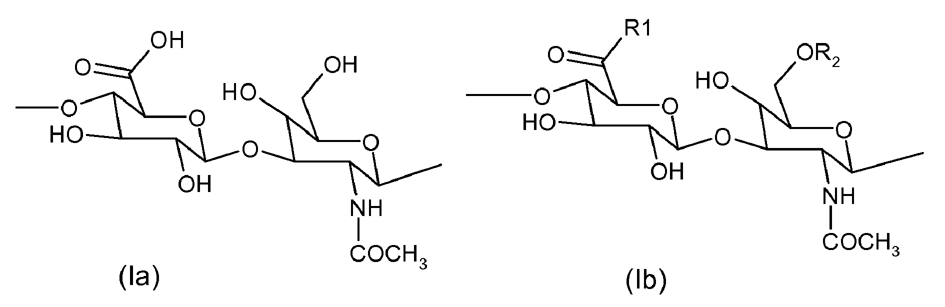 worin:
- R1 für OH oder einen Rest -NH-R'-Z steht,
- R2 für H oder einen Rest -C(O)-NH-R'-Z steht,
wobei:
- R' ein zweiwertiger, linearer oder verzweigter C1-C18-, insbesondere C2-C14-, sogar C4-C10-Alkylrest oder eine Einfachbindung ist;
- Z ein Rest mit der Formel (I) ist: 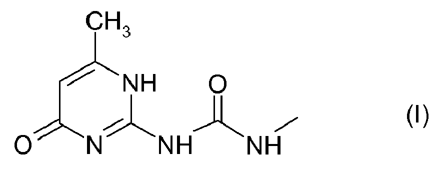
wobei als vereinbart gilt, dass mindestens einer der Reste R1 und R2 einen Rest Z umfasst,
der Funktionalisierungsgrad der Verbindung im Bereich zwischen 1 und 30 % liegt,
die Verbindung ein mittleres Molekulargewicht Mw im Bereich zwischen 50.000 und 2.000.000 aufweist.

## Claims

1. Cosmetic or dermatological composition comprising a hyaluronic acid compound formed by the repetition of a hydrophilic disaccharide unit, in which sodium D-glucuronate and/or D-glucuronic acid is connected to N-acetylglucosamine via alternating β1-4 and β1-3 glycoside bonds or β1-4 glycoside bonds alone, comprising units (Ia) and units (Ib), or one of its salts: 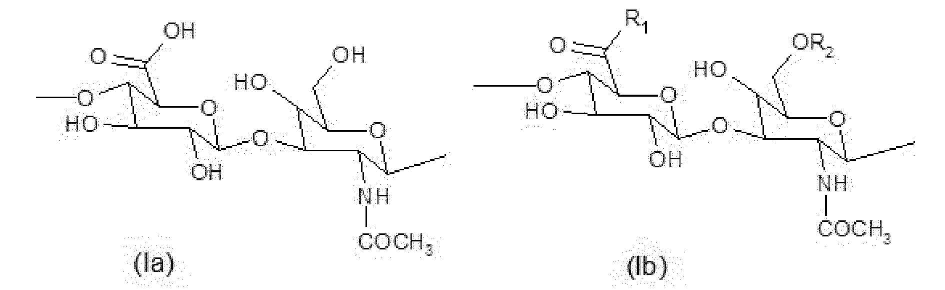 in which:
- R₁ represents OH or an -NH-R'-Z radical,
- R₂ represents H or a -C(O)-NH-R'-Z radical,
with:
- R' a linear or branched, divalent C₁-C₁₈, in particular C₂-C₁₄, indeed even C₄-C₁₀, alkyl radical; or a single bond;
- Z a radical of formula (I): 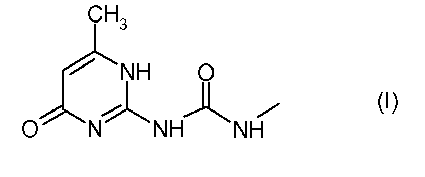
it being understood that at least one of the R₁ and R₂ radicals comprises a Z radical, the degree of functionalization of the compound being between 1% and 30%, the compound having an average molecular weight Mw of between 50 000 and 2 000 000.

2. Composition according to the preceding claim, in which the compound is present, alone or as mixtures, in an amount ranging from 0.1% to 3% by weight, relative to the total weight of the composition, in particular from 0.5% to 2% by weight, indeed even from 0.8% to 1.5% by weight.

3. Composition according to either of the preceding claims, which is provided in the form of a product for caring for or making up the skin of the face or body, the lips, the nails, the eyelashes, the eyebrows or the hair; of an aftershave gel or lotion; of a depilatory cream; of a body or hair hygiene composition, such as a shower gel, a shampoo, a soap or a cleansing bar; of a hair product, in particular for cleaning, styling, caring for, treating, repairing, dyeing or fixing the hair, such as a hair-setting lotion, a styling cream or gel, a dyeing composition, a hair-restructuring lotion, a permanent-wave composition, or a lotion or gel for combating hair loss; or of an oral composition.

4. Composition according to one of the preceding claims, comprising at least one ingredient chosen from silicone fatty substances, such as silicone oils, gums and waxes, and also non-silicone fatty substances, such as oils, pastes and waxes of vegetable, mineral, animal and/or synthetic origin; water, a C₂-C₆ alcohol; glycols, such as propylene glycol, ketones; thickeners, emulsifiers, surfactants, gelling agents, cosmetic active agents, fragrances, fillers, colourants, moisturizing agents, vitamins or polymers.

5. Cosmetic treatment method, especially for making up, caring for, cleaning or dyeing keratin materials, especially the skin of the body or face, the lips, the nails, the hair and/or the eyelashes, comprising applying to the keratin materials a cosmetic composition as defined in one of Claims 1 to 4.

6. Method according to Claim 5, **characterized in that** it is a cosmetic treatment method for reducing the signs of ageing of the skin and/or mucous membranes, in particular for reducing wrinkles and fine lines and/or for restoring the volume thereof to the face or body.

7. Hyaluronic acid compound formed by the repetition of a hydrophilic disaccharide unit, in which sodium D-glucuronate and/or D-glucuronic acid is connected to N-acetylglucosamine via alternating β1-4 and β1-3 glycoside bonds or β1-4 glycoside bonds alone, comprising units (Ia) and units (Ib), or one of its salts: 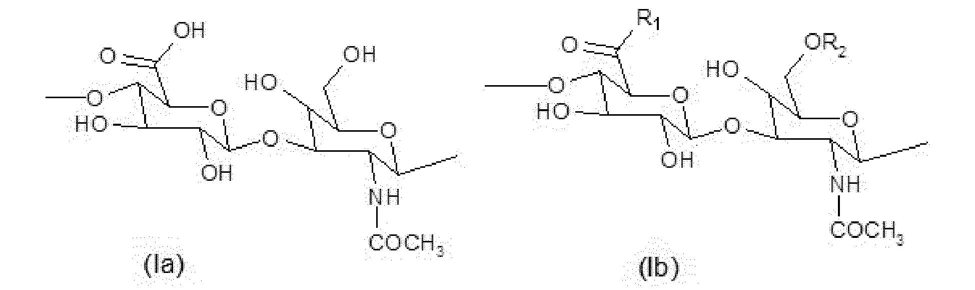 in which:
- R₁ represents OH or an -NH-R'-Z radical,
- R₂ represents H or a -C(O)-NH-R'-Z radical,
with:
- R' a linear or branched, divalent C₁-C₁₈, in particular C₂-C₁₄, indeed even C₄-C₁₀, alkyl radical; or a single bond;
- Z a radical of formula (I): 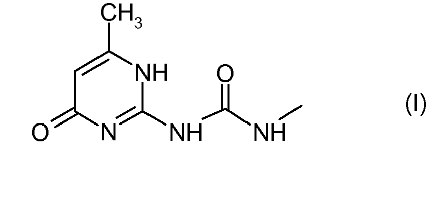
it being understood that at least one of the R₁ and R₂ radicals comprises a Z radical, the degree of functionalization of the compound being between 1% and 30%, the compound having an average molecular weight Mw of between 50 000 and 2 000 000.
